# EUROPÄISCHE PATENTSCHRIFT

(11) **EP 2 340 770 B1**
(45) Veröffentlichungstag und Bekanntmachung des Hinweises auf die Patenterteilung: **18.03.2020**
(21) Anmeldenummer: 10016186.8
(22) Anmeldetag: 30.12.2010
(51) Int. Cl.: A61B 17/00, A61B 17/11

(54) **Medizinisches Implantat zum Verschliessen von Gefässöffnungen**
Medical implant for closing vascular openings
Implant médical destiné à la fermeture d'ouvertures vasculaires

(30) Priorität: 30.12.2009 DE 102009060770
(43) Veröffentlichungstag der Anmeldung: 06.07.2011
(73) Patentinhaber: Bentley InnoMed GmbH, 72379 Hechingen (DE)
(72) Erfinder: Obradovic, Milisav, 79539 Lörrach (DE)
(74) Vertreter: Hoefer & Partner Patentanwälte mbB

(56) Entgegenhaltungen:
- EP-A2- 1 917 913
- WO-A2-2009/137755
- US-A1- 2003 139 819
- US-A1- 2004 143 277
- US-A1- 2005 038 470
- US-A1- 2006 116 710
- US-A1- 2008 077 180
- US-A1- 2008 249 562
- US-A1- 2009 228 038

## Beschreibung

Die Erfindung betrifft ein medizinisches Implantat zum Verschließen von Gefäßöffnungen und/oder Shunt-Verbindungen zwischen Körpergefäßen, das in einem kontrahierten Zustand minimal-invasiv positionierbar und in einen expandierten Zustand überführbar ist, in dem es zwei voneinander beabstandete, im Durchmesser erweiterte Dichtelemente aufweist, die die Gefäßöffnung oder Shunt-Verbindung auf beiden Seiten abdichten.

Solche auch als Okkluder bezeichnete Implantate sind bereits bekannt. So beschreibt beispielsweise die EP 0 808 138 B1 einen Okkluder, der aus Metallgewebe aus miteinander verflochtenen Metalllitzen gefertigt ist. Der Okkluder wird mithilfe eines Katheters an die gewünschte Stelle im vaskulären System eines Patienten platziert und in den expandierten Zustand gebracht. Mithilfe des Okkluders lassen sich Gefäße eines Patienten verschließen, beispielsweise septale Defekte, wie etwa Defekte des Vorhofseptums. Auch ein persistierender Duktus (PDR) oder ein Ventrikelseptumdefekt können mit Okkludern behandelt werden. Ein weiteres, ähnliches Okklusionsinstrument ist aus der DE 10 2005 053 958 A1 bekannt. Auch dieses Okklusionsinstrument besteht aus einem Metallgewebe mit zwei schirmartigen Dichtelementen, wobei im implantierten Zustand eines der Dichtelemente auf der einen Seite der Gefäßöffnung und das andere Dichtelement auf der gegenüberliegenden Seite der Gefäßöffnung an der Gefäßwand anliegt und die zu schließende Gefäßöffnung abdichtet.

Bei den bekannten Okkludern können Membranen zur Abdichtung vorgesehen sein. Dies bedeutet, dass die Membranen von einem Metallgeflecht umgeben sind, sodass zum einen das Metallgeflecht unmittelbar an der Gefäßwand anliegt und zum anderen in das Gefäß ragt. Es ist erstrebenswert, dass die Dichtelemente von Gewebe überwachsen werden. Dabei ist es jedoch nachteilig, wenn das zu überwachsende Implantat strukturiert ist, wenn also ein Metallgeflecht mit entsprechend vielen Zwischenräumen überwachsen werden muss. Das Metallgeflecht begünstigt auch eine unerwünschte Thrombusbildung.

Aus Dokumenten US 2008/0249562 A1, US 2008/077180A2, US 2003/0139819 A1, US 2006/0116710 A1 und US2009/0228038 A1 sind Implantate zum Verschluss von Gefäßöffnungen oder Shunt-Verbindungen bekannt mit zwei, im expandierten Zustand, voneinander beabstandeten Dichtelementen, wobei die Dichtelemente jeweils einen Überzug aufweisen. Aus beispielsweise US 2006/0116710 A1 ist auch bekannt, dass die Dichtelemente im zentralen Bereich über Stege miteinander verbunden sind und eine Außenseite eines Überzugs unterbrechungsfrei ist. US 2008/0249562 A1, US 2008/077180A2, US 2006/0116710 A1 und US2009/0228038 A1 weisen auch Überzüge für Dichtelemente auf die aus mehreren Teilen bestehen.
Aufgabe der vorliegenden Erfindung ist es deshalb, ein Implantat bereitzustellen, welches besonders einfach und schnell mit Körpergewebe bedeckt werden kann. Gelöst wird diese Aufgabe erfindungsgemäß durch ein medizinisches Implantat zum Verschließen von Gefäßöffnungen und/oder Shunt-Verbindungen zwischen Körpergefäßen entsprechend den Merkmalen des Anspruchs 1, besondere Ausführungsformen sind in den Unteransprüchen angegeben. Das Implantat ist in einem kontrahierten Zustand minimal-invasiv positionierbar und in einen expandierten Zustand überführbar, in dem es zwei voneinander beabstandete, im Durchmesser erweiterte Dichtelemente aufweist, die die Gefäßöffnung der Shunt-Verbindung auf beiden Seiten abdichten, wobei zumindest ein Dichtelement einen Überzug aufweist der haubenartig ausgebildet ist. Dies bedeutet, dass der expandierte, formgebende Teil eines Dichtelements, insbesondere ein Drahtgeflecht bzw. eine strukturierte Oberfläche eines Dichtelements nicht mehr exponiert ist. Stege und Strukturen der Dichtelemente sind durch einen Überzug abgedeckt. Dadurch entsteht eine glattere Oberfläche, sodass das Überwachsen des medizinischen Implantats und im Besonderen der Dichtelemente erleichtert und beschleunigt wird. Außerdem wird eine Thrombosegefahr reduziert.

Dadurch dass der Überzug haubenartig ausgebildet ist, kann sichergestellt werden, dass Strukturen und Stege zumindest weitestgehend von dem Überzug abgedeckt sind und somit strukturierte Oberflächen, die mit Gewebe überwachsen werden müssen, minimiert werden. Wenn der Überzug haubenartig ausgebildet ist, ist es nicht unbedingt notwendig, den Überzug separat zu befestigen. Dadurch, dass der Überzug ein Dichtelement, d. h. dessen formgebendes Teil weitestgehend umgibt, kann er formschlüssig daran gehalten sein.

Auf besonders einfache Art und Weise kann der Überzug hergestellt werden, wenn er aus zwei Teilen besteht, die miteinander verbunden sind, insbesondere vernäht, verklebt oder verschweißt sind. Dabei kann vorgesehen sein, dass die beiden Teile des Überzugs beidseits eines formgebenden Teils, insbesondere eines Geflechts oder dergleichen, des Dichtelements in Anlage gebracht und anschließend miteinander verbunden werden. Dadurch ist es nicht notwendig, ein Geflecht oder dergleichen später im fertig gestellten Überzug zu positionieren.

Weitere Vorteile ergeben sich, wenn die Dichtelemente Stege oder Drähte aufweisen, und zumindest der Überzug eines Dichtelements mit zumindest einem Steg oder Draht verbunden ist, insbesondere damit vernäht ist. Damit kann zum einen sichergestellt werden, dass sich ein Überzug nicht unbeabsichtigt von einem Dichtelement löst. Zum anderen kann dadurch sichergestellt werden, dass der Überzug bezüglich des den Überzug tragenden Teils des Dichtelements nach der Expansion des Dichtelements richtig positioniert ist. Weiterhin wird dadurch sichergestellt, dass der Überzug zusammen mit dem den Überzug tragenden, expandierbaren Teil des Dichtelements zum Einführen bzw. Positionieren des medizinischen Implantats komprimiert wird. In diesem Zusammenhang ist es besonders vorteilhaft, wenn zumindest ein Steg oder Draht eines Dichtelements zumindest eine ösenartige Öffnung aufweist. Ein Faden oder dergleichen kann durch die ösenartige Öffnung gefädelt werden und dann mit dem Überzug verbunden werden. Dadurch ist die Position des Befestigungselements, also des Fadens, an dem Draht oder Steg festgelegt. Die Befestigung kann nicht verrutschen.

Gemäß der Ausgestaltung der Erfindung ist vorgesehen, dass die einer Gefäßwand abgewandte Seite (Außenseite) eines Überzugs unterbrechungsfrei ist. Eine solche Überzugseite weist keinerlei Struktur auf, sodass ein besonders gutes und zuverlässiges Überwachsen mit Gewebe ermöglicht wird und Thrombosebildung vermieden wird.

Weiterhin ist vorgesehen, dass die einer Gefäßwand abgewandte Seite des anderen Überzugs eine Öffnung aufweist, durch die das Implantat mit einem Positionierwerkzeug verbindbar ist. Dadurch wird eine notwendige Verbindungsmöglichkeit des Implantats mit einem Positionierwerkzeug geschaffen und gleichzeitig eine Unterbrechung eines Überzugs minimiert, indem nur an der Stelle eine Öffnung vorgesehen ist, wo das Positionierwerkzeug an dem Implantat angreifen muss.

Gemäß einer Weiterbildung kann vorgesehen sein, dass der Überzug im Bereich der Öffnung mit dem den Überzug tragenden Teil des zugeordneten Dichtelements verbunden ist. Dadurch wird sichergestellt, dass das Verbindungsteil für das Positionierwerkzeug stets durch die Öffnung zugänglich ist und nicht versehentlich die Öffnung diesen Teil des Implantats verdeckt und somit keine Verbindung mit einem Positionierwerkzeug mehr möglich ist. Außerdem wird durch diese Maßnahme der Überzug ortsfest am Implantat festgelegt.

Besonders vorteilhaft ist es, wenn der Überzug aus Kunststoff, etwa Teflon ® oder ePTFE und/oder textilem Material ausgebildet ist. Derartig beschaffene Überzüge sind sehr glatt, so dass ein zuverlässiges Überwachsen mit Gewebe ermöglicht wird. Außerdem wird die Thrombusbildung reduziert.

Die Dichtelemente können über starre oder elastische Elemente miteinander verbunden sein. Je nach Anwendungsfall kann eine geeignete Verbindung der Dichtelemente gewählt werden, insbesondere über elastische Stege.

Besonders vorteilhaft ist es, wenn die Dichtelemente in axialer Richtung des Implantats durch die Überzüge komprimiert gehalten sind. Dies bedeutet, dass die Dichtelemente sehr flach sind. Sie ragen somit nur geringfügig in ein Gefäß und behindern daher nur geringfügig den Fluss von Körperflüssigkeiten in den Gefäßen.

Das Implantat kann aus einem rohrförmigen Körper durch Einbringen von Wandöffnungen mindestens im Bereich der im expandierten Zustand einen erweiterten Durchmesser aufweisenden Dichtelemente hergestellt sein.

Die erfindungsgemäßen Implantate werden also vorzugsweise nicht aus Geweben oder Netzen hergestellt, die zuvor in relativ aufwändigen Verfahren aus dünnen Fäden oder Drähten gefertigt werden. Stattdessen wird das Implantat aus einem massiven rohrförmigen Körper gefertigt, der zumindest an den Stellen, die im expandierten Zustand die Dichtelemente bilden, mit Wandöffnungen versehen wird. Durch das Einbringen der Wandöffnungen ist es möglich, die Dichtelemente mit den erweiterten Durchmessern herzustellen, beispielsweise indem der rohrförmige Körper gestaucht wird. Die Wandöffnungen lassen sich vorzugsweise durch Laserschneiden in den rohrförmigen Körper einbringen. Dieses Verfahren ist hochpräzise und relativ preisgünstig. Der gegenseitige Abstand der Wandöffnungen kann auch derart gewählt werden, dass die dazwischen stehen bleibenden Wandstege des rohrförmigen Körpers dicker sind als die Fäden oder Litzen der bekannten Okkluder. Dadurch lassen sich erfindungsgemäße Implantate mit einer höheren Formstabilität als die bekannten Okkluder herstellen.

Vorzugsweise können die Wandöffnungen dabei schlitzförmig ausgebildet sein. Diese Form der Wandöffnungen ermöglicht eine leichte Verformbarkeit des rohrförmigen Körpers, sodass die im expandierten Zustand einen erweiterten Durchmesser aufweisenden Dichtelemente einfach herstellbar sind. Weitere Vorteile ergeben sich, wenn mehrere, parallel zueinander ausgerichtete Wandöffnungen vorgesehen sind. Auch diese Maßnahme erleichtert die Verformbarkeit des Implantats. Der gegenseitige Abstand der Wandöffnungen und ihre Verteilung über den Umfang des rohrförmigen Körpers kann entsprechend den gewünschten Eigenschaften des Implantats gewählt werden. Die Konfiguration der Wandöffnungen bestimmt die Eigenschaften der Dichtelemente.

Bei einer bevorzugten Variante können sich die Wandöffnungen im Wesentlichen parallel zur Längsachse des Implantats erstrecken. Dadurch sind sie einfach in den rohrförmigen Körper einzubringen und gewährleisten außerdem ein leichtes Überführen des Implantats in den expandierten Zustand. Die Wandöffnungen können jedoch auch in einem spitzen Winkel zur Längsachse des Implantats verlaufen oder sich spiralförmig über den Umfang des Implantats erstrecken.

Weitere Vorteile ergeben sich, wenn das Implantat aus einer Formgedächtnislegierung, insbesondere aus Nitinol gefertigt ist. Bei der Verwendung solcher Materialien lässt sich der expandierte Zustand des Implantats fixieren. Nitinol weist zugleich federnde Eigenschaften auf, sodass das Implantat zum Einführen in den Körper des Patienten in den kontrahierten Zustand gebracht und beispielsweise mittels eines Katheters eingeführt werden kann. Am Zielort kann der Katheter zurückgezogen werden, wodurch das Implantat die abgespeicherte expandierte Form annimmt, in dem sie in der Lage ist, die Gefäßöffnung oder die Shunt-Verbindung abzudichten.

Alternativ zur Verwendung einer Formgedächtnislegierung kann auch ein Formgedächtnispolymer zur Herstellung des erfindungsgemäßen Implantats verwendet werden. Polymere haben ebenfalls hervorragende Formgedächtniseigenschaften und lassen sich noch einfacher verformen als Nitinol.

Zu Herstellung des Implantats können Wandöffnungen in den rohrförmigen Körper nicht nur in den Bereichen der Dichtelemente, sondern auch in einem zentralen Bereich zwischen den Dichtelementen eingebracht werden. Wird das Implantat dergestalt ausgebildet, so ist es möglich, durch Stauchen und/oder Verwinden den Durchmesser des Implantats und den gegenseitigen Abstand der Dichtelemente zu verändern. Damit lässt sich eine gute Zentrierung des Implantats in der Gefäßöffnung oder Shunt-Verbindung erzielen. Gleichzeitig werden die Überzüge der Dichtelemente gegen die Gefäßwand in Anlage gebracht, d. h. eine gute Dichtwirkung des Implantats erreicht.

Wie die bekannten Okkluder kann auch das erfindungsgemäße Implantat vorzugsweise mithilfe eines Katheters oder einer Wendel im Körper positionierbar sein. Zusätzlich kann das erfindungsgemäße Implantat durch einen Führungsdraht geführt und positioniert werden. Mithilfe des Katheters oder der Wendel kann das Implantat mit hoher Genauigkeit durch das vaskuläre System des Patienten bewegt und am gewünschten Einsatzort positioniert werden. Für die Befestigung des Katheters oder der Wendel kommen unterschiedliche Möglichkeiten in Betracht. Bei einer besonders einfachen Ausgestaltung ist der Katheter oder die Wendel mittels einer Rastverbindung mit einem der Enden des Implantats verbindbar und in einer nicht erfindungsgemäßen Ausgestaltung könnte eine Schraubverbindung oder ein Bajonettverschluss vorliegen zur Verbindung mit einem Ende des Implantats.

In einem nicht erfindungsgemäßen Beispiel kann nur ein einziger Überzug für das Implantat vorgesehen sein, der beide Dichtelemente umfasst, insbesondere das gesamte Implantat umgibt. Dieser Überzug kann auch im zentralen Bereich an dem rohrförmigen Körper befestigt sein. Dazu können zwei haubenartige Überzüge über den Dichtelementen im zentralen Bereich miteinander verbunden sein. Der oder die Überzüge der Dichtelemente sind, sofern notwendig, vorzugsweise über Nähte, mit den Dichtelementen verbunden wie auch untereinander. Aus mehreren Teilen bestehende Überzüge sind vorzugsweise im peripheren Bereich miteinander und mit den Dichtelementen vernäht.

Nachfolgend werden bevorzugte Ausführungsbeispiele eines erfindungsgemäßen Implantats anhand der Zeichnung näher beschrieben.

Im Einzelnen zeigen:
- Fig. 1: eine Seitenansicht auf ein erfindungsgemäßes Implantat im expandierten Zustand;
- Fig. 2: eine perspektivische Darstellung des Implantats aus Fig. 1 ohne Überzüge;
- Fig. 3: eine perspektivische Ansicht eines rohrförmigen Körpers zur Herstellung eines erfindungsgemäßen Implantats;
- Fig. 4: eine Darstellung zur Erläuterung der Befestigung eines Überzugs an einem Steg;
- Fig. 5: eine alternative, nicht erfindungsgemäße Ausführungsform eines Implantats;
- Fig. 6: eine Detaildarstellung der Fig. 5;
- Fig. 7: eine Schnittdarstellung durch den zentralen Bereich eines Implantats;
- Fig. 8: zeigt eine Ausführungsform eines erfindungsgemäßen Implantats mit Überzug in (a) expandierter Form und (b) in komprimierter Form;
- Fig. 9: ein erfindungsgemäßes Implantat (a) von der Außenseite des distalen Schirms und (b) von der Außenseite des proximalen Schirms;
- Fig. 10: ein Applikationssystem mit dem erfindungsgemäßen Implantat;
- Fig. 11: Details der Verbindung des Applikationssystems von Fig. 10;
- Fig. 12: Details der Formschlussverbindung und der Klick-Verbindung des Applikationssystems von Fig. 10 und
- Fig. 13: ein erfindungsgemäß geeignetes Applikationssystem mit Applikationsgriff.

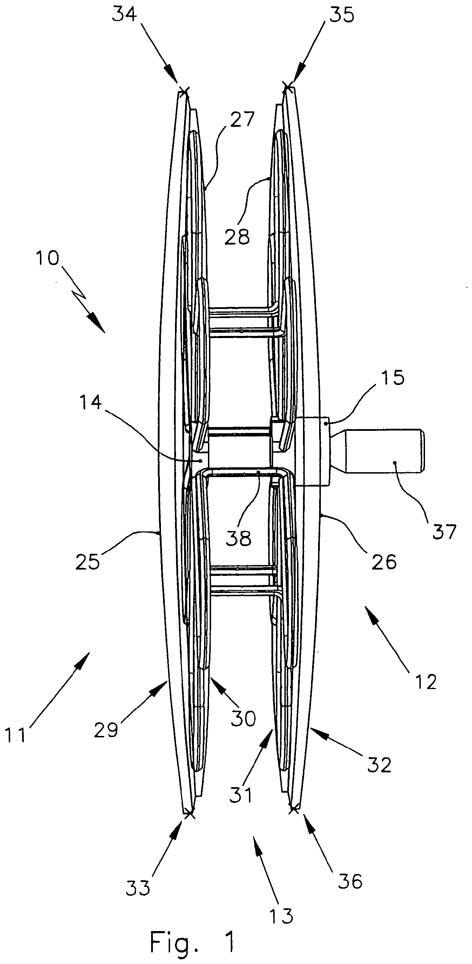

Das in Fig. 1 dargestellte Implantat 10 weist zwei Dichtelemente 11, 12 auf, die gegenüber einem dazwischen liegenden zentralen Bereich 13 sowie den Endbereichen 14, 15 einen deutlich erweiterten Durchmesser aufweisen. Die Dichtelemente 11, 12 weisen jeweils einen haubenartigen Überzug 25, 26 auf. Im implantierten Zustand ragt der zentrale Bereich 13 durch die zu verschließende Gefäßöffnung bzw. Shunt-Verbindung, während die beiden Dichtelemente 11, 12 beidseitig der Gefäßöffnung bzw. Shunt-Verbindung und die Seiten 27, 28 der Überzüge 25, 26 an der Gefäßwand anliegen. Die Überzüge 25, 26 sind im Ausführungsbeispiel mit jeweils zwei Hälften 29, 30; 31, 32 ausgebildet, die miteinander vernäht sind, was an den Stellen 33 - 36 angedeutet ist. Zumindest die Hälften 30, 31, also die der Gefäßwand zugewandten Seiten (Innenseiten) 27, 28 der Überzüge 25, 26 sind als flüssigkeitsdichte Membranen ausgebildet, die dafür sorgen, dass das Implantat 10 die Gefäßöffnung oder die Shunt-Verbindung gegen den Durchtritt von Flüssigkeiten, insbesondere von Blut abdichtet.

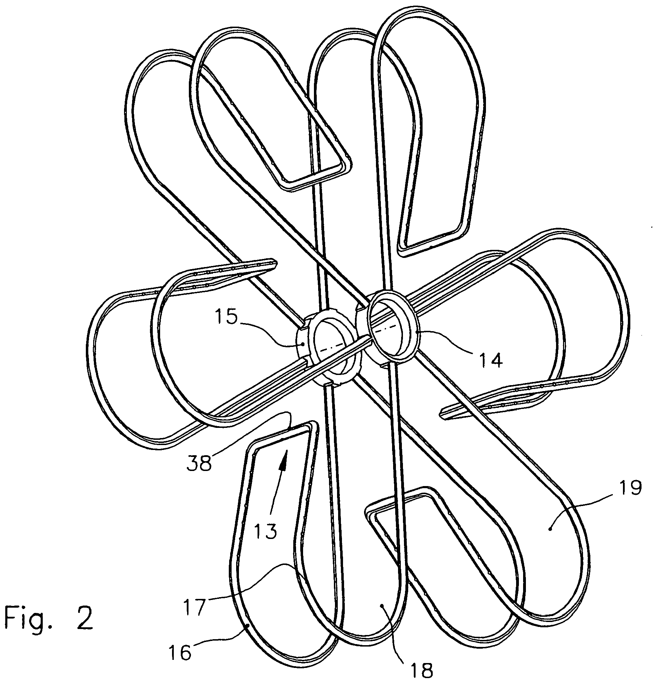

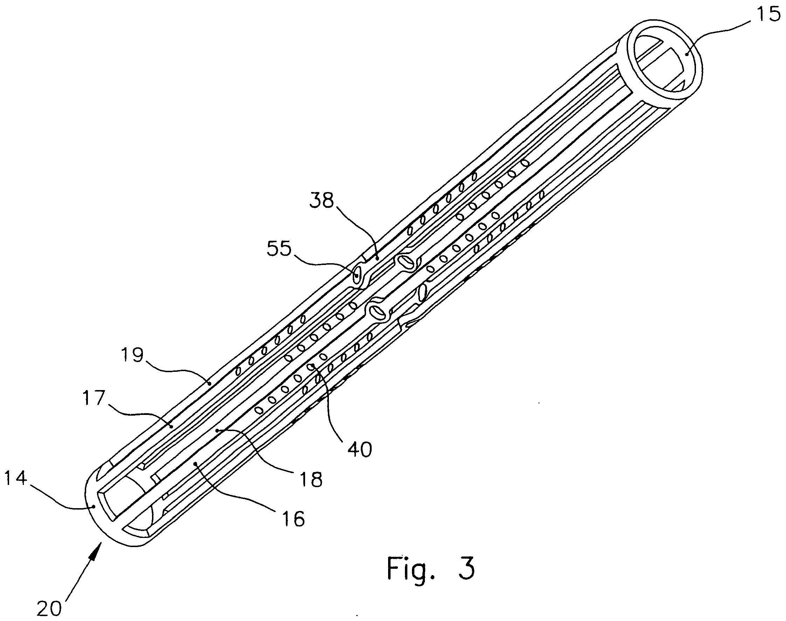

Die Stege 16, 17 (Fig. 2) der Dichtelemente 11, 12, der zentrale Bereich 13 sowie die beiden Endbereiche 14, 15 sind einteilig ausgebildet. Während die Bereiche 14 und 15 von massiven Rohrabschnitten gebildet sind, sind die Stege 16, 17 bogenförmig in einer Ebene angeordnet und die gleichmäßig über den Umfang der Dichtelemente 11, 12 verteilt, wie insbesondere auch Fig. 2 zeigt, die das Implantat 10 (ohne Überzüge) in perspektivischer Ansicht zeigt. Die beiden Dichtelemente 11, 12 sind im zentralen Bereich 13 über Stege 38 miteinander verbunden. Die (Metall-) Stege 16, 17 entstehen durch Einschneiden von schlitzförmigen Wandöffnungen 18, 19 in einen rohrförmigen Körper 20, wie er beispielsweise in Fig. 3 gezeigt ist. Durch Stauchen des rohrförmigen Körpers 20 in seiner Längsrichtung biegen sich dann die Metallstege 16, 17 auf. Diese Metallstege 16, 17 tragen die Überzüge 25, 26 und stellen den formgebenden Teil der (expandierten) Dichtelemente 11, 12 dar. Der Zustand, der in Fig. 1 gezeigt ist, entspricht dem expandierten Zustand des Implantats 10. Wird das Implantat 10 dabei aus einem Material mit Formengedächtnis hergestellt, so kann diese expandierte Form im Material gespeichert werden.

Mit der Bezugsziffer 37 ist ein Teil eines Positionierwerkzeugs gezeigt, mit dem das Implantat 10 positioniert werden kann und das lösbar mit dem Implantat 10 verbindbar ist.

Zum Einführen des Implantats 10 wird das Implantat 10 dagegen wieder in seinen kontrahierten Zustand gebracht, der demjenigen, der in Fig. 3 gezeigt ist, entspricht. Im kontrahierten Zustand sind die Implantate 10 rohrförmig und lassen sich dadurch leicht mit einem Katheter oder einer Wendel minimal-invasiv über das vaskuläre System an den gewünschten Einsatzort verbringen. Ist das Implantat 10 positioniert, so kann der expandierte, gespeicherte Zustand des Implantats 10 aktiviert werden, wodurch das Implantat 10 seine abdichtende Wirkung entfalten kann.

Das in Fig. 3 gezeigte Implantat 10 wird aus einem massiven rohrförmigen Körper 20 durch Einbringen von schlitzförmigen Wandöffnungen, insbesondere mithilfe eines Lasers hergestellt. Es wurden jedoch nicht nur im Bereich der Dichtelemente 11, 12 schlitzförmige Wandöffnungen eingebracht, sondern auch im zentralen Bereich 13. Als massive Rohrstücke bleiben nur die Endbereiche 14, 15.

Der zentrale Bereich 13 wird durch Stege 38 gebildet, die im Wesentlichen parallel zur Längsachse des Implantats verlaufen. Die Ebenen der Dichtelemente 11, 12 stehen senkrecht zur Längsachse. Die Stege sind elastisch verformbar.

Die Implantate 10 können beispielsweise aus Nitinol oder einer anderen Metalllegierung mit Formengedächtnis oder aber auch aus einem anderen Formgedächtnispolymer hergestellt sein. Die hier nicht gezeigten Überzüge im Bereich der Dichtelemente 11, 12 können mit einer glatten Oberfläche, vorzugsweise aus ePTFE, gefertigt sein, da sich dieses Material durch eine sehr gute Körperverträglichkeit auszeichnet.

Das in den Figuren gezeigte Implantat 10 wurde durch Einbringen von schlitzförmigen Wandöffnungen 18, 19 hergestellt, die parallel zur Längsachse des rohrförmigen Körpers 20 verlaufen. Dies ist jedoch nicht zwingend. Die Öffnungen 18, 19 können mit der Längsachse des rohrförmigen Körpers 20 auch einen Winkel bilden, wodurch auch die Stege 16, 17 Schlaufen bilden, deren Anfangs- und Endpunkte in tangentialer Richtung versetzt zueinander sind. Auch spiralförmig verlaufende Wandöffnungen 18, 19 oder andere Verläufe sind möglich.

Die in der Fig. 1 gezeigten Dichtelemente 11, 12 können alternativ auch einen formgebenden Teil aus einem Drahtgeflecht aufweisen. Um den Überzug 26 festzulegen, kann dieser im Endbereich 15 des Implantats 10 mit den Metallstegen 17 verbunden, insbesondere vernäht sein.

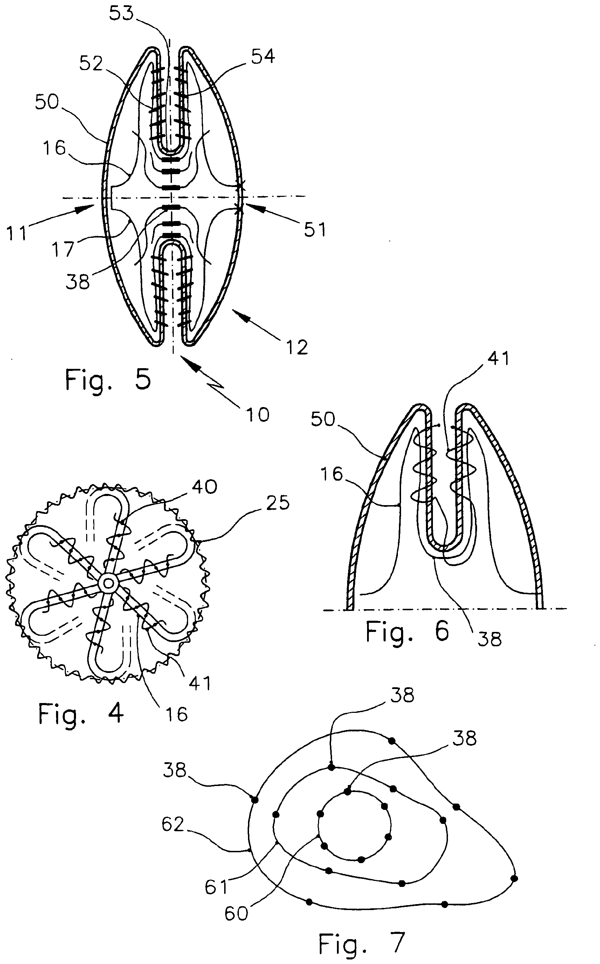

Die Figuren 1 und 4 zeigen ein bevorzugtes Ausführungsbeispiel, wie ein Überzug 25, 26 mit einem Metallsteg 16, 17 verbunden werden kann. Hierzu weist der Metallsteg 16, 17 vorzugsweise ösenartige Öffnungen 40 auf, die beispielsweise auch durch Laserschneiden hergestellt werden können. Durch die ösenartige Öffnung 40 ist ein Faden 41 gefädelt, durch den der Überzug 25, 26 an dem Steg 16, 17 gehalten wird. In den gezeigten Ausführungsbeispielen liegen die Stege 16, 17 in einer Ebene im Wesentlichen parallel zur Gefäßwand bzw. senkrecht zur Längsachse des Implantats 10. Bei Ausführungsformen mit schlaufenartigen Stegen 16, 17 können die Dichtelemente 11, 12 durch die Überzüge 25, 26 in axialer Richtung des Implantats komprimiert, d. h. flach gehalten werden. Wenn der Überzug 25, 26 mit den Stegen 16, 17 der Dichtelemente 11, 12 verbunden ist, insbesondere mit diesen vernäht ist, werden die Überzüge 25, 26 auch zusammengezogen und komprimiert, sodass die komprimierte Form des Implantats 10 nur unwesentlich größer ist als in der Fig. 3 dargestellt, wo kein Überzug eingezeichnet ist. Dadurch lässt sich das Implantat 10 besonders einfach positionieren. Werden die Dichtelemente 11, 12 expandiert, so werden auch die Überzüge 25, 26 mit expandiert bzw. ausgefaltet, sodass die Metallstege 16, 17 der Dichtelemente 11, 12 von innen an den Überzügen 25, 26 anliegen.

Die Überzüge 25, 26 weisen vorzugsweise eine glatte Oberfläche auf. Dies ist insbesondere in der Fig. 1 zu sehen, wo die Hälfte 29 des Überzugs 25 unterbrechungsfrei ausgebildet ist und somit keinerlei Oberflächenstrukturen aufweist. Dies ermöglicht ein besonders gutes Überwachsen mit Gewebe. Wie sich anhand der übrigen Ausgestaltung der Überzüge 25, 26 erkennen lässt, sind Durchbrechungen der Überzüge 25, 26 auf ein Minimum und somit das Notwendigste reduziert.

In der Fig. 5 ist schematisch eine nicht erfindungsgemäße Ausführungsform gezeigt, bei der ein einziger Überzug 50 für das gesamte Implantat 10 vorgesehen ist, der die Metallstege 16, 17 beider Dichtelemente 11, 12 umgibt und sich auch über den zentralen Bereich 13 erstreckt. Dieser Überzug 50 weist nur eine Öffnung 51 auf.

Der Überzug 50 ist mit den Stegen 16, 17 der Dichtelemente 11, 12 und den Stegen 38 vernäht, was durch die Nähte 52, 53, 54 angedeutet ist.

Zu diesem Zweck weisen die Stege 38 Öffnungen 55 auf. In der vergrößerten Darstellung der Fig. 6 ist zu sehen, dass ein einziger Faden 41 zum Verbinden des Überzugs 50 mit aneinander anschließenden Stegen 16, 17, 38 verwendet werden kann, der abwechselnd durch Überzug und Öffnungen 40, 55 verläuft.

Fig. 7 zeigt, wie der zentrale Bereich 13 durch entsprechende Formung des Implantats 10 an verschiedene Septumdefekte angepasst werden kann. Die Bezugsziffern 60 bis 62 zeigen die Formen für unterschiedliche Defektgrößen. Die Stege 38 müssen dabei nicht auf einer Kreislinie angeordnet sein.

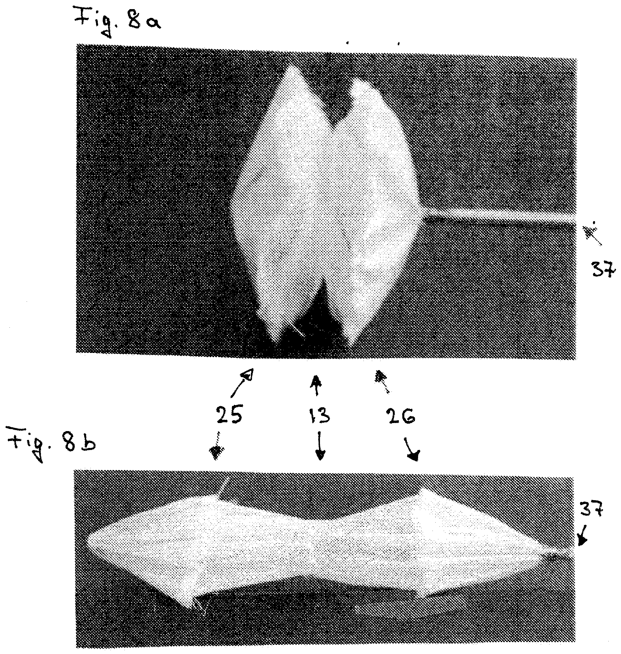

Fig. 8 zeigt eine vergrößerte Fotografie einer bevorzugten Ausführungsform eines erfindungsgemäßen Implantats in expandiertem Zustand (Fig. 8a) und in komprimiertem Zustand (Fig. 8b). Zu erkennen sind die Dichtelemente mit den jeweiligen haubenförmigen Überzügen 25 und 26 und der dazwischen liegende zentrale Bereich 13. Dabei bezeichnet die Bezugsziffer 25 den haubenförmigen Überzug im Bereich des distalen Dichtelements (distal zum Positionierwerkzeug) 37 und die Bezugsziffer 26 den Überzug des proximalen Dichtelements. Der Überzug ist aus mehreren Teilen zusammengesetzt, die im Bereich der Peripherie der Dichtelemente miteinander vernäht sind wie auch im zentralen Bereich 13. Das Positionierwerkzeug 37 (teilweise dargestellt) ist im Bereich des proximalen Dichtelements mit dem Implantat verkoppelt; in diesem Bereich hat der Überzug eine Öffnung.

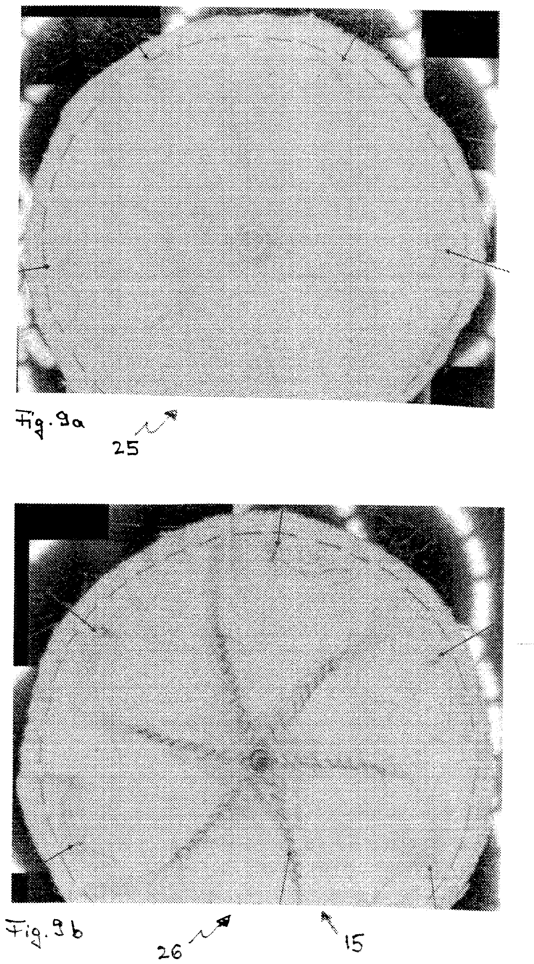

Fig. 9a zeigt das erfindungsgemäße Implantat von Fig. 8 von der Stirnseite, d. h. die Außenseite bzw. Stirnfläche des Überzugs 25 auf dem distalen Dichtelement 11. Die Pfeile bezeichnen die Lage der 6 bogen- bzw. schlaufenförmig ausgebildeten Stege 16. Deutlich zu erkennen ist die umlaufende Rundnaht, die die beiden Teile 29, 30 des Überzugs 25 miteinander peripher verbindet. In jedem Fall ist es bevorzugt, dass die Außenseite des Überzugs am distalen Schirm wenig Nähte und damit eine sehr glatte Oberfläche aufweist, um das Einwachsen zu begünstigen. Nichts desto weniger kann es sinnvoll sein, eine zusätzliche Vernähung des Überzugs 25 insbesondere auf dessen Innenseite mit den Bögen bzw. Streben des Dichtelements 11 vorzusehen.

Fig. 9b zeigt den Überzug des proximalen Dichtelements 12 von der Außenseite/Stirnseite. Zu erkennen ist hier ebenfalls die umlaufende Rundnaht, die die beiden Hälften 31, 32 des Überzugs miteinander verbindet. Zusätzlich ist der Überzug mit den auswärtsweisenden, radialverlaufenden Stegen 17 des Dichtelements 12 vernäht. Deutlich zu erkennen ist die zentrale Öffnung 51 im Überzug 26 mit dem Endbereich 15 des proximalen Dichtelements 12.

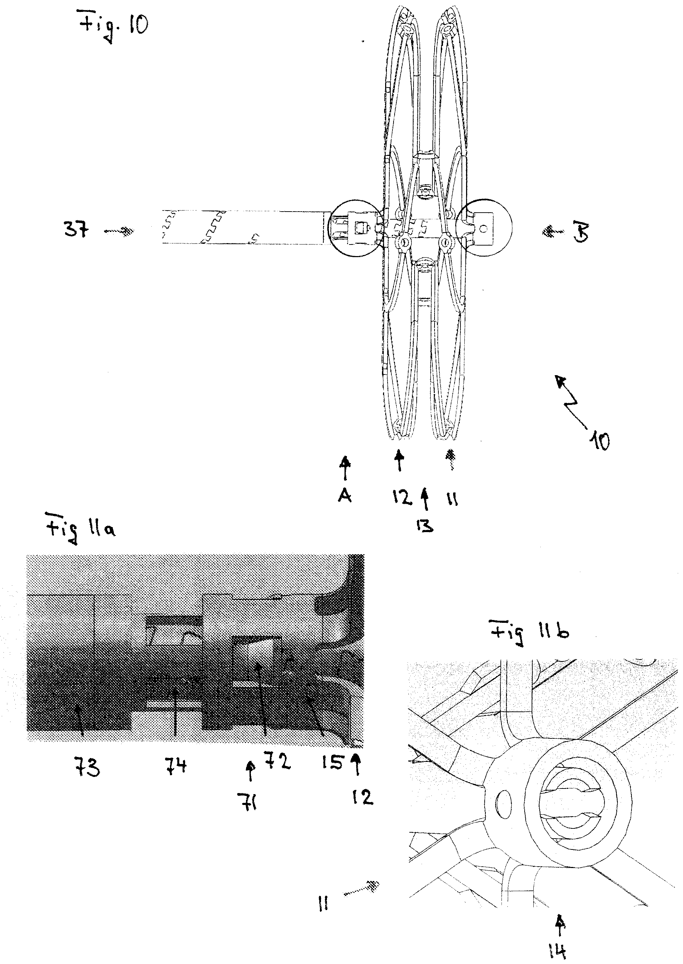

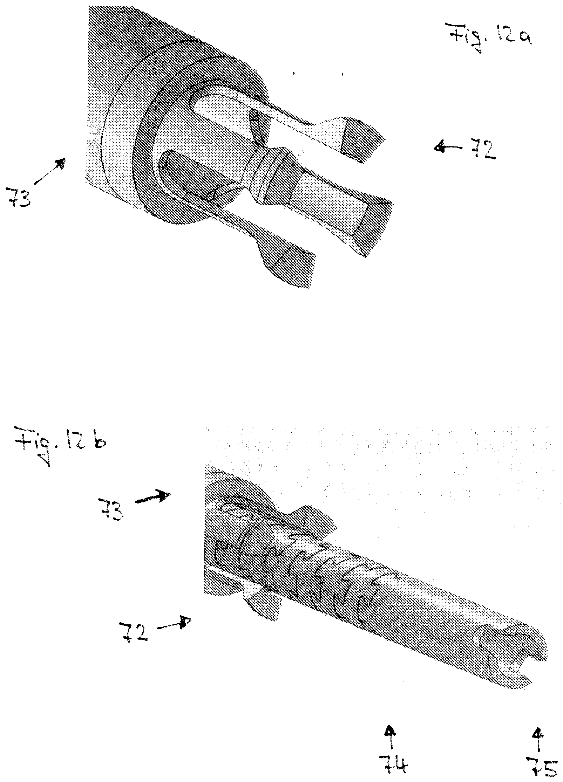

Fig. 10 zeigt ein erfindungsgemäßes Implantat ohne Überzug mit dem dazugehörigen Positionierwerkzeug 37. Das Positioniersystem ist in den Details A und B in Fig.11 und Fig. 12 dargestellt.

Das Positioniersystem 37 ist lösbar über die Endstücke 14, 15 mit dem Implantat 1 verbunden und besteht im Prinzip aus einem Innenrohr und einem Außenrohr sowie Kopplungselementen. Das Implantat 1 ist in aufgespanntem, expandiertem Zustand gezeigt; deutlich zu erkennen ist das proximale Dichtelement 12, das distale Dichtelement 11 sowie der dazwischen liegende zentrale Bereich 13, der einen größeren Innendurchmesser hat als die Endstücke 14, 15 jedoch einen deutlich kleineren als die Dichtelemente 11, 12 in expandiertem Zustand.

Fig. 11a zeigt das Detail A aus Fig. 10 mit dem rohrförmigen Endstück 15 und dem daraus abzweigenden Dichtelement 12. Das Endstück 15 hat insgesamt 4 Ausnehmungen 71 in die elastische Elemente oder Zapfen 72 des Außenrohrs 73 eingreifen. Innerhalb des Außenrohrs 73 verläuft ein Innenrohr 74, das die Zapfen 72 aufspreizt und in den Ausnehmungen 71 festlegt. Dieses Innenrohr 74 ist innerhalb des Außenrohrs 73 beweglich und kann zurückgezogen werden, der Gestalt, dass bei Rückzug über die Position der Zapfen 72 hinaus diese aus den Ausnehmungen 71 ausschnappen und das Implantat freigeben.

Fig. 11b zeigt das Detail B aus Fig. 10 mit dem Endbereich 14 des distalen Dichtelements 11. Zu erkennen sind die Streben des Dichtelement 11 sowie der Verlauf des Innenrohrs 74, das in den am Endstück 14 angeordneten Okkluderstift eingreift bzw. dieses in einer Ausnehmung aufnimmt. Mit dem Innenrohr kann das Implantat 10 aus der gestreckten Transportform in die expandierte Okklusionsform gebracht werden, was eine präzise Positionierung im Körper des Patienten erlaubt. Bei abgeschlossener Positionierung wird das Innenrohr 74 vorsichtig zurückgezogen, so dass es aus dem Okkluderstift ausrastet, sodass das Implantat 10 dann (mit dem nicht gezeigten Überzug) in der Endposition verbleibt. Bei weiterem Rückzug des Innenrohrs 74 rasten die Zapfen 72 aus den Ausnehmungen 71 aus und ziehen sich zurück, sodass das Implantat 10 freigesetzt ist.

Fig. 12 zeigt Details der Formschlussverbindung und der Klick-Verbindung von Fig. 10. In Fig. 12a ist die Formschlussverbindung für den Anschluss an das proximale Endstück 15 und den proximalen Schirm 12 dargestellt mit dem Außenkatheter 73 und den Zapfen oder Rastelementen 72, hier dargestellt in aufgespreiztem Zustand. Fig. 12b zeigt den Endbereich des Außenrohrs 73 mit eingeführtem Innenrohr 74, das durch den Bereich der Rastelemente 72 hindurchführt und am distalen Ende eine Ausnehmung 75 aufweist, die den Okkluderstift des Endstücks 14 rastend in sich aufnimmt.

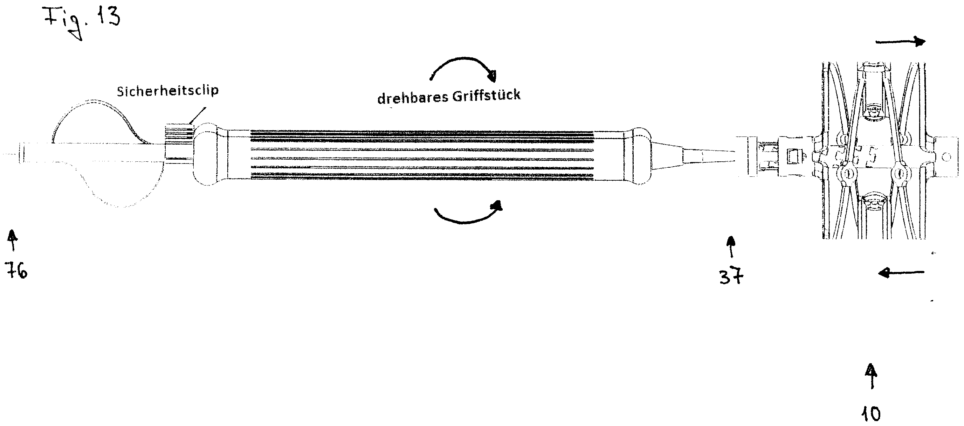

Fig. 13 zeigt das Applikationssystem insgesamt mit dem aufgespannten Implantat 10 (ohne Überzug), dem Applikationssystem sowie dem dazugehörigen Handhabungsgriff, der ein präzises Strecken und Positionieren des Implantats durch Drehung erlaubt. Der eingebaute Sicherheitsclip muss vor Entfernung des Innenrohrs und damit Applikation des Implantats entfernt werden; ein unabsichtliches Implantieren ist damit nicht möglich. Das drehbare Griffstück ist so eingestellt, dass eine komplette Drehung die Streckung bzw. Expansion des Implantats über die gesamte bewegliche Länge ermöglicht. Am Ende des Applikationssystems ist ein Führungsdraht 76 zu erkennen, der dazu dient, das Implantat samt Positionierungsystem im Patienten an den Einsatzort zu manövrieren.

Es versteht sich, dass das erfindungsgemäße Implantat mit seinem Applikationssystem im Innern eines üblichen Platzierungskatheters manövriert und geführt werden kann.

## Patentansprüche

1. Medizinisches Implantat (10) zum Verschließen von Gefäßöffnungen und/oder Shunt-Verbindungen zwischen Körpergefäßen, das in einem kontrahierten Zustand minimal-invasiv positionierbar und in einen expandierten Zustand überführbar ist, in dem es zwei voneinander beabstandete, im Durchmesser erweiterte Dichtelemente (11, 12) aufweist, die die Gefäßöffnung oder Shunt-Verbindung auf beiden Seiten abdichten, wobei die Dichtelemente (11, 12) jeweils einen Überzug (25, 26) aufweisen, **dadurch gekennzeichnet, dass** die Dichtelemente (11, 12) im zentralen Bereich (13) über Stege (38) miteinander verbunden sind, welche im Wesentlichen parallel zur Längsachse des Implantats (10) verlaufen und die Überzüge (25, 26) jeweils aus zwei Teilen (29 bis 32) bestehen, die miteinander verbunden sind, wobei die einer Gefäßwand abgewandte Seite (29) eines Überzugs (25) unterbrechungsfrei ist und die einer Gefäßwand abgewandte Seite (32) des anderen Überzugs (26) eine Öffnung aufweist, durch die das Implantat (10) mit einem Positionierwerkzeug (37) verbindbar ist und die Überzüge (25, 26) der beiden Dichtelemente (11, 12) im zentralen Bereich (13) miteinander verbunden sind, wobei das Positionierwerkzeug (37) aus einem Außenrohr (73) und einem im Außenrohr (73) beweglichen Innenrohr (74) besteht, wobei das Außenrohr (73) an seinem distalen Ende über mindestens zwei bevorzugt vier Rastelemente (72) und das Innenrohr (74) an seinem distalen Ende über mindestens eine Ausnehmung (75) verfügen und das medizinische Implantat (10) über ein distales (14) und ein proximales (15) rohrförmiges Endstück verfügt, an denen die Dichtelemente (11, 12) befestigt sind, und sich im distalen Endstück (14) ein Stift befindet, in den die distale Ausnehmung (75) des Innenrohrs (74) einrasten kann, und sich im proximalen Endstück (15) Ausnehmungen (71) befinden, in die die Rastelemente (72) des Außenrohrs (73) eingreifen können, wobei die distale Ausnehmung (71) des Innenrohrs (74) durch Rückzug des Innenrohrs (74) aus dem Stift des distalen Endstücks (14) ausrastet und die Rastelemente (72) des Außenrohrs (73) durch Zurückziehen des Innenrohrs (74) über den Punkt der Ausnehmungen (71) aus den Ausnehmungen (71) im proximalen Endstück (15) ausrasten.

2. Implantat nach Anspruch 1, **dadurch gekennzeichnet, dass** das Implantat (10) aus einem rohrförmigen Körper (20) durch Einbringen von Wandöffnungen (18, 19) mindestens im Bereich der im expandierten Zustand einen erweiterten Durchmesser aufweisenden Dichtelemente (11,12) hergestellt ist.

3. Implantat nach einem der vorhergehenden Ansprüche, **dadurch gekennzeichnet, dass** der jeweilige Überzug (25, 26) aus zwei Teilen (29 - 32) besteht, die miteinander vernäht, verklebt oder verschweißt sind.

4. Implantat nach einem der vorhergehenden Ansprüche, **dadurch gekennzeichnet, dass** die Dichtelemente (11, 12) Stege aufweisen, und der Überzug (25, 26) zumindest eines Dichtelements (11, 12) mit zumindest einem Steg (16, 17) verbunden ist, insbesondere damit vernäht ist.

5. Implantat nach Anspruch 4, **dadurch gekennzeichnet, dass** zumindest ein Steg (16, 17) eines Dichtelements (11, 12) zumindest eine ösenartige Öffnung (40) aufweist.

6. Implantat nach Anspruch 1, **dadurch gekennzeichnet, dass** der Überzug (26) im Bereich der Öffnung mit dem zugeordneten Dichtelement (12) über Nähte verbunden ist.

7. Implantat nach einem der vorhergehenden Ansprüche, **dadurch gekennzeichnet, dass** der Überzug (25, 26) aus Kunststoff, Teflon, und/oder textilem Material ausgebildet ist.

8. Implantat nach einem der vorhergehenden Ansprüche, **dadurch gekennzeichnet, dass** die Dichtelemente (11, 12) über starre oder elastische Stege (38) verbunden sind.

9. Implantat nach einem der vorhergehenden Ansprüche, **dadurch gekennzeichnet, dass** durch Stauchen und/oder Verwinden des Implantats (10) die Überzüge (25, 26) der beiden Dichtelemente (11, 12) gegen die die Gefäßöffnung oder Shunt-Verbindung begrenzende Gefäßwand in Anlage bringbar sind.

10. Implantat nach einem der vorhergehenden Ansprüche, **dadurch gekennzeichnet, dass** es mithilfe eines Katheters oder einer Wendel als Positionierungswerkzeug im Körper positionierbar ist.

11. Implantat nach Anspruch 10, **dadurch gekennzeichnet, dass** der Katheter oder die Wendel an einem der rohrförmigen Enden (14, 15) des Implantats (10) durch Einrasten befestigbar ist.

## Claims

1. A medical implant (10) for closing vascular openings and/or shunt connections between body vessels, which can be positioned minimally invasively in a contracted state and converted into an expanded state, in which it has two spaced-apart, diameter-expanded sealing elements (11, 12), which seal the vessel opening or shunt connection on both sides, wherein the sealing elements (11, 12) respectively have a coating (25, 26), **characterised in that** the sealing elements (11, 12) are connected to each other in the central area (13) via webs (38), which essentially run in parallel to the longitudinal axis of the implant (10), and the coatings (25, 26) respectively consist of two parts (29 to 32), which are connected to each other, wherein the side (29) of a coating (25) facing away from a vessel wall is uninterrupted and the side (32) of the other coating (26) facing away from a vessel wall has an opening, through which the implant (10) is connectable with a positioning tool (37) and the coatings (25, 26) of the two sealing elements (11, 12) are connected to each other in the central area (13), wherein the positioning tool (37) consists of an outer tube (73) and an inner tube (74) movable in the outer tube (73), wherein the outer tube (73) has at least two, preferably four, latching elements (72) at its distal end and the inner tube (74) has at least one recess (75) at its distal end and the medical implant (10) has a distal (14) and a proximal (15) tubular end piece, to which the sealing elements (11, 12) are fastened, and a pin is located in the distal end piece (14), into which the distal recess (75) of the inner tube (74) can latch, and recesses (71) are located in the proximal end piece (15), into which the latching elements (72) of the outer tube (73) can engage, wherein the distal recess (71) of the inner tube (74) unlatches from the pin of the distal end piece (14) by retraction of the inner tube (74) and the latching elements (72) of the outer tube (73) unlatch from the recesses (71) in the proximal end piece (15) by retracting the inner tube (74) over the point of the recesses (71).

2. The implant according to claim 1, **characterised in that** the implant (10) is produced from a tubular body (20) by introducing wall openings (18, 19) at least in the region of the sealing elements (11, 12) having an expanded diameter in the expanded state.

3. The implant according to any one of the preceding claims, **characterised in that** the respective coating (25, 26) consists of two parts (29 - 32), which are sewn, glued or welded to each other.

4. The implant according to any one of the preceding claims, **characterised in that** the sealing elements (11, 12) have webs, and the coating (25, 26) of at least one sealing element (11, 12) is connected to at least one web (16, 17), in particular sewn thereto.

5. The implant according to claim 4, **characterised in that** at least one web (16, 17) of a sealing element (11, 12) has at least one eye-like opening (40).

6. The implant according to claim 1, **characterised in that** the coating (26) is connected to the allocated sealing element (12) via seams in the area of the opening.

7. The implant according to any one of the preceding claims, **characterised in that** the coating (25, 26) is formed from plastic, Teflon and/or textile material.

8. The implant according to any one of the preceding claims, **characterised in that** the sealing elements (11, 12) are connected via rigid or elastic webs (38).

9. The implant according to any one of the preceding claims, **characterised in that**, by upsetting and/or twisting the implant (10), the coatings (25, 26) of the two sealing elements (11, 12) can be brought into contact with the vessel wall delimiting the vascular opening or shunt connection.

10. The implant according to any one of the preceding claims, **characterised in that** it can be positioned in the body by means of a catheter or a helix as the positioning tool.

11. The implant according to claim 10, **characterised in that** the catheter or the helix can be fastened to one of the tubular ends (14, 15) of the implant (10) by latching it in.

## Revendications

1. Implant médical (10) servant à fermer des ouvertures de vaisseau et/ou des liaisons de pontage entre des vaisseaux corporels, qui peut être positionné de manière invasive au minimum dans un état contracté et peut être transféré dans un état expansé, dans lequel il présente deux éléments d'étanchéité (11, 12) espacés l'un de l'autre, au diamètre élargi, qui étanchéifient sur les deux côtés l'ouverture de vaisseau ou la liaison de pontage, dans lequel les éléments d'étanchéité (11, 12) présentent respectivement un revêtement (25, 26),
**caractérisé en ce que**
les éléments d'étanchéité (11, 12) sont reliés l'un à l'autre dans la zone centrale (13) par l'intermédiaire d'entretoises (38), lesquelles s'étendent de manière sensiblement parallèle par rapport à l'axe longitudinal de l'implant (10) et les revêtements (25, 26) sont constitués respectivement de deux parties (29 à 32), qui sont reliées l'une à l'autre, dans lequel le côté (29), opposé à une paroi de vaisseau, d'un revêtement (25) est sans interruption et le côté (32), opposé à une paroi de vaisseau, de l'autre revêtement (26) présente une ouverture, à travers laquelle l'implant (10) peut être relié à un outil de positionnement (37) et les revêtements (25, 26) des deux éléments d'étanchéité (11, 12) sont reliés les uns aux autres dans la zone centrale (13), dans lequel l'outil de positionnement (37) est constitué d'un tuyau extérieur (73) et d'un tuyau intérieur (74) mobile dans le tuyau extérieur (73), dans lequel le tuyau extérieur (73) dispose au niveau de son extrémité distale d'au moins deux, de préférence quatre, éléments d'enclenchement (72) et le tuyau intérieur (74) dispose au niveau de son extrémité distale d'au moins un évidement (75) et l'implant médical (10) dispose de pièces d'extrémités tubulaires distale (14) et proximale (15), au niveau desquelles les éléments d'étanchéité (11, 12) sont fixés, et dans la pièce d'extrémité distale (14) se trouve une tige, dans laquelle l'évidement distal (75) du tuyau intérieur (74) peut s'enclencher, et dans la pièce d'extrémité proximale (15) se trouvent des évidements (71), avec lesquels les éléments d'enclenchement (72) du tuyau extérieur (73) peuvent venir en prise, dans lequel l'évidement distal (71) du tuyau intérieur (74) se désenclenche par retrait du tuyau intérieur (74) de la tige de la pièce d'extrémité distale (14) et les éléments d'enclenchement (72) du tuyau extérieur (73) se désenclenchent par enlèvement du tuyau intérieur (74) des évidements (71) dans la pièce d'extrémité proximale (15) par l'intermédiaire du point des évidements (71).

2. Implant selon la revendication 1, **caractérisé en ce que** l'implant (10) est fabriqué à partir d'un corps (20) tubulaire en pratiquant des ouvertures de paroi (18, 19) au moins dans la zone des éléments d'étanchéité (11, 12) présentant dans l'état expansé un diamètre élargi.

3. Implant selon l'une quelconque des revendications précédentes, **caractérisé en ce que** le revêtement (25, 26) respectif est constitué de deux parties (29 - 32), qui sont cousues, collées ou soudées l'une à l'autre.

4. Implant selon l'une quelconque des revendications précédentes, **caractérisé en ce que** les éléments d'étanchéité (11, 12) présentent des entretoises, et le revêtement (25, 26) d'au moins un élément d'étanchéité (11, 12) est relié à au moins une entretoise (16, 17), en particulier y est cousu.

5. Implant selon la revendication 4, **caractérisé en ce qu'**au moins une entretoise (16, 17) d'un élément d'étanchéité (11, 12) présente au moins une ouverture (40) de type œillet.

6. Implant selon la revendication 1, **caractérisé en ce que** le revêtement (26) est relié dans la zone de l'ouverture à l'élément d'étanchéité (12) associé par l'intermédiaire de coutures.

7. Implant selon l'une quelconque des revendications précédentes, **caractérisé en ce que** le revêtement (25, 26) est réalisé à partir de matière plastique, de téflon et/ou d'un matériau textile.

8. Implant selon l'une quelconque des revendications précédentes, **caractérisé en ce que** les éléments d'étanchéité (11, 12) sont reliés par l'intermédiaire d'entretoises (38) rigides ou élastiques.

9. Implant selon l'une quelconque des revendications précédentes, **caractérisé en ce que** les revêtements (25, 26) des deux éléments d'étanchéité (11, 12) peuvent être amenés en appui contre la paroi de vaisseau délimitant l'ouverture de vaisseau ou la liaison de pontage par enfoncement et/ou torsion de l'implant (10).

10. Implant selon l'une quelconque des revendications précédentes, **caractérisé en ce qu'**il peut être positionné dans le corps à l'aide d'un cathéter ou d'une hélice en tant qu'outil de positionnement.

11. Implant selon la revendication 10, **caractérisé en ce que** le cathéter ou l'hélice peut être fixé au niveau d'une des extrémités (14, 15) tubulaires de l'implant (10) par enclenchement.
